# EUROPEAN PATENT SPECIFICATION

(11) **EP 1 594 499 B1**
(45) Date of publication and mention of the grant of the patent: **08.04.2009**
(21) Application number: 03814711.2
(22) Date of filing: 11.12.2003
(51) Int. Cl.: A61K 31/4439, A61K 45/00, A61K 47/02, A61P 1/04, A61P 29/00

(54) **DOSAGE FORMS CONTAINING A PROTON PUMP INHIBITOR, A NSAID, AND A BUFFER**
DARREICHUNGSFORMEN ENTHALTEND EINEN PROTONPUMPHEMMER, EINEN NSAID UND EINEN PUFFER
FORMES DOSIFIÉES CONTENANT UN IPP, UN AINS ET UN TAMPON

(30) Priority: 20.12.2002 US 325338
(43) Date of publication of application: 16.11.2005
(73) Proprietor: TAP Pharmaceutical Products, Inc., Lake Forest, Illinois 60045 (US)
(72) Inventor: TANEJA, Rajneesh, Libertyville, IL 60048 (US)
(74) Representative: Modiano, Micaela Nadia
(86) International application number: PCT/US2003/039349
(87) International publication number: WO 2004/060372

(56) References cited:
- WO-A-02/22108
- US-A1- 2002 192 299
- US-B1- 6 489 346
- DATABASE BIOSIS [Online] BIOSCIENCES INFORMATION SERVICE, PHILADELPHIA, PA, US; December 2001 (2001-12) WILSON INGALILL ET AL: "Management of gastroduodenal ulcers and gastrointestinal symptoms associated with nonsteroidal anti-inflammatory drug therapy: A summary of four comparative trials with omeprazole, ranitidine, misoprostol, and placebo" Database accession no. PREV200200162984 XP002279060 & CURRENT THERAPEUTIC RESEARCH, vol. 62, no. 12, December 2001 (2001-12), pages 835-850, ISSN: 0011-393X

## Description

### Technical Field

The present invention relates to non-steroidal anti-inflammatory drugs (NSAIDs) and in particular, relates to dosage forms containing a proton pump inhibitor (PPI), a buffer, and an NSAID.

### Background

NSAIDs are one of the most commonly prescribed medicines. NSAIDs are commonly used to treat rheumatoid arthritis, osteoarthritis, mild to moderate pain and a host of other conditions.

While NSAIDs are effective for a variety of indications, side effects associated with their use detract from their usefulness, particularly on a long term basis. NSAIDs operate by inhibiting an enzyme known as cyclooxygenase (COX). The COX enzyme has two forms known as COX-1 and COX-2. COX-1 is an enzyme that produces a product that helps protect the lining of the stomach, and COX-2 is involved in the process of inflammation. Hence, when NSAIDs are taken, they reduce inflammation but, at the same time, inhibit an enzyme partly responsible for protecting the lining of the stomach. As a result, NSAIDs are associated with ulcers and gastro-intestinal bleeding.

Aspirin is an NSAID that is renowned for its curative properties. Aspirin is given for pain and is also commonly given to cardiac and stroke patients because of the benefits these patients derive from aspirin. In fact, studies have shown that aspirin reduces the risk of strokes and heart attacks. Aspirin, however, is not different from other NSAIDs in terms of its effects on the gastrointestinal tract. Hence, although its properties are well known, many patients in need of long term aspirin therapy cannot receive it due its propensity to cause gastrointestinal bleeding. As a result, patients are deprived of a beneficial therapy.

There is therefore a need for a means to allow patients to enjoy the many and well documented benefits of NSAIDs without experiencing their detrimental side effects.

### Summary of the Invention

The present invention provides dosage forms, methods, and formulations that permit patients to receive the benefits of NSAID therapy without experiencing the gastrointestinal side effects associated with these medications. Formulations provided herein comprise a non-enterically coated dosage form comprising a proton pump inhibitor; a buffer; and an NSAID. These formulations can be given to patients in need of NSAID therapies for any of the well known conditions for which NSAIDs are prescribed. Hence, also provided herein is the use of said formulations in methods for protecting the gastrointestinal tract from the effects of NSAIDs, such methods generally comprise providing the formulation described above.

### Detailed Description of the Invention

As previously mentioned, formulations provided herein comprise a PPI, a buffer, and an NSAID. The dosage form containing these components does not need to be enterically coated. It was discovered that an NSAID and a PPI could co-exist in a single dosage form without the need for an enteric coating covering the PPI. PPI's are notoriously unstable in acidic environments and are commonly covered in an enteric coating to protect them from such environments, including those typically found in the stomach. NSAIDs are typically acidic and it was therefore surprising to find that a PPI was stable (or could be sufficiently stabilized) in an unprotected form when combined with the NSAID.

As a result, many benefits are derived from the dosage form provided herein. For example, manufacturing costs are significantly reduced because there is no need to enterically coat the PPI and formulate the enterically coated PPI with the other ingredients in such a way as to not disturb the integrity of the protective enteric coating. Additionally, the PPI is protected in the stomach due to the ability of the buffer to increase the pH of the stomach to a level where the PPI is not degraded. Moreover, due to the lack of an enteric coating the PPI enters the intestine in a form that is freely absorbed. Typically, an enteric coated PPI is not readily available in the intestine because the enteric coating must first be dissolved. As a result, the onset time of action for the PPI is reduced. Further, the buffer provides immediate relief from gastric irritation and protects the stomach from local irritation sometimes caused simply by the presence of an NSAID in the stomach. Accordingly, NSAIDs can now be delivered in a single dosage form with a minimum of gastrointestinal side effects typically associated with these beneficial compounds.

PPI's are compounds that inhibit the hydrogen-potassium adenosine triphosphate enzyme system. This enzyme system is variously referred to as proton pumps and it is the proton pumps that ultimately provide the stomach with acid. Many PPIs are commercially available and are commonly prescribed for gastro oesophageal reflux disease (GERD) as well as other indications. Any compound having PPI activity can be used in the present formulation and examples of such PPI's include lansoprazole, omeprazole, rabeprazole, and pantoprazole, as well as, for example, prodrugs and enantiomers of any of the above.

NSAIDs are described above and are also well known. Any NSAID, or enantiomer or prodrug of an NSAID, can be employed in the formulation provided herein. Examples of NSAIDs include but are not limited to diclofenac, misoprostol, diflunisal, etodolac, fenoprofen, flurbiprofen, ibuprofen, indomethacin, ketoprofen, ketorolac, meclofenamate, mefenamic, meloxicam, nabumetone, naproxen, oxaprozin, piroxicam, salsalate, sulindac, tolmetin, and aspirin.

Formulations of the invention also include a buffer. The term buffer as used herein means any compound or combination of compounds that increase the pH of the environment in which they are dissolved or dispersed. Buffers generally can be broken into two categories based upon their solubility. Both categories of buffer separately, or in combination, can be employed in the formulation. "Water-soluble buffers" typically have a solubility in water of at least 1 gm in 100 ml, preferably at least 1 gm in 75 ml, and more preferably at least 1 gm in 30 ml. Examples of water-soluble buffers include, but are not limited to meglumine, sodium bicarbonate, sodium carbonate, sodium citrate, calcium gluconate, disodium hydrogen phosphate, dipotasium hydrogen phosphate, tripotasium phosphate, sodium tartarate, sodium acetate, calcium glycerophosphate, and preferably tromethamine, magnesium oxide or any combination of the foregoing. "Water-insoluble buffers" typically have a solubility in water less than 1 gm in 1,000 ml, preferably less than 1 gm in 5,000 ml, and more preferably less than 1 gm in 10,000 ml. Examples of water-insoluble buffers include, but are not limited to magnesium hydroxide, aluminum hydroxide, dihydroxy aluminum sodium carbonate, calcium carbonate, aluminum phosphate, aluminum carbonate, dihydroxy aluminum amino acetate, magnesium oxide, magnesium trisilicate, magnesium carbonate, and combinations of the foregoing.

PPI's, NSAIDs, and buffers are typically used in therapeutically effective amounts. The phrase "therapeutically effective amount" as used herein means a sufficient amount of, for example, the PPI, NSAID, buffer, or formulation necessary to treat the desired disorder, at a reasonable benefit/risk ratio applicable to any medical treatment. As with other pharmaceuticals, it will be understood that the total daily usage of a pharmaceutical composition of the invention will be decided by a patient's attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time administration, route of administration, and rate of excretion of the specific compound employed; the duration of the treatment; drugs used in combination or coincidental with the specific compound employed; and other factors known to those of ordinary skill in the medical arts. For example, it is well within the skill of the art to start doses of the compound at levels lower than required to achieve the desired therapeutic effect and to gradually increase the dosage until the desired effect is achieved.

Formulations of the invention are administered and dosed in accordance with sound medical practice, taking into account the clinical condition of the individual patient, the site and method of administration, scheduling of administration, and other factors known to medical practitioners.

The typical daily pharmaceutically effective amount of the compounds administered to a patient in single or divided doses typically range from about 0.1 to about 200 mg/kg body weight, preferably from about 0.25 to about 100 mg/kg body weight.

In terms of the buffer, while the buffer serves a pharmaceutical purpose in the formulation, it also serves to raise the pH of the stomach sufficiently to protect the acid labile PPI. Preferably, therefore, the buffer can elevate the pH of 50 ml of simulated gastric fluid (as described below) above 7 within 20 minutes, more preferably within 15 minutes and most preferably within 10 minutes or less. Additionally, the buffer preferably can maintain the pH of simulated gastric fluid in simulated gastric conditions (also described below) above 3 for 30 minutes, preferably above 3 for 60 minutes, and more preferably above 3 for 90 minutes. Preferably combinations of soluble and insoluble buffers are used to achieve the above.

Simulated gastric fluid ("SGF") typically is made by dissolving 2.0 gm of sodium chloride and 3.2 gm of purified pepsin (derived from porcine stomach mucosa) having an activity of 800 to 2500 units per mg of protein, in 7.0 ml of hydrochloric acid (HCl) and sufficient water to make a 1000 mL solution. Solutions of SGF typically have a pH of less than 3. In cases where the SGF does not have a pH of less than 3, the solution can be pH'ed, as is well known in the art, to a preferred pH range of between 1.5 and 2.0 with additional HCl. "Simulated gastric conditions" can be achieved by filling a container such as a beaker with 100 ml of SGF and adding additional SGF (typically between 10 ml to 35 ml of fresh SGF) to a container as additional components (such as the formulation or buffers described herein) are added to the initial SGF.

The amount and ratio of the water-soluble buffer and water-insoluble buffer in a formulation generally does not depend upon the amount of the PPI in the formulation and may vary widely to achieve a rapid and sustained pH increase sufficient to protect a PPI from degradation. Exact amounts of the buffer or buffers employed is a matter of choice for those skilled in the art which can be determined empirically using SGF and simulated gastric conditions. For example, different amounts and proportions of the neutralizers may be tested in various amounts simulated gastric fluid and conditions to arrive at a desired effect. Most preferably, the quantity of water-soluble buffer in the formulation is between 50 mg and 1000 mg, preferably between 100 mg and 600 mg, and more preferably between 300 mg and 500 mg. The quantity of water-insoluble buffer in the formulation is typically between 100 mg and 1000 mg, preferably between 250 mg and 750 mg, and more preferably between 250 mg and 600 mg.

Of course, any of the components in the formulation including PPIs, NSAIDs, buffers, or other excipients used in the formulation should be pharmaceutically acceptable. The phrase "pharmaceutically acceptable" as used herein includes moieties or compounds that are, within the scope of sound medical judgment, suitable for use in contact with the tissues of humans and lower animals without undue toxicity, irritation, allergic response, and the like, and are commensurate with a reasonable benefit/risk ratio.

Formulations provided herein may also contain other well known pharmaceutically acceptable ingredients such as carriers, diluents, excipients, fillers and the like. The formulations provided herein can be administered in either a solid or liquid dosage form. Solid dosage forms of the invention for oral administration generally are fabricated in a similar manner to the tablets of the examples below. Similarly, liquid dosage forms of the invention for oral administration can be pharmaceutically acceptable emulsions, solutions, suspensions, syrups, and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, goundnut, corn, germ, olive, castor, and/or sesame oils), glycerol, tetrahydrofurfuryl alcohol, polyethylene glycols and fatty acid esters of sorbitan and mixtures thereof.

Besides inert diluents, oral compositions of the invention may also include adjuvants such as wetting agents, emulsifying and suspending agents, sweetening agents, flavoring agents, and/or perfuming agents.

Compositions of the invention can be manufactured by utilizing an acid-labile and/or acid-stable pharmaceutical compound in the form of granules and/or powder. Alternatively, micronized acid-labile pharmaceutical compositions can be used in place of the granules or powder. Micronization is utilized in order to produce a particle having a smaller diameter in relationship to the granules. Since the dissolution rate of acid-labile pharmaceutical compositions of the invention is generally directly proportional to, among other factors, the surface area of the composition particle, a reduction in particle size increases the amount of exposed surface area and, thus, increases the dissolution rate.

Although micronization results in increased exposed surface area causing particle aggregation, which can negate the benefit of micronization and is an expensive manufacturing step, micronization of the proton pump inhibitor does present a significant benefit of increasing the dissolution rate of relatively water-insoluble drugs, e.g. omeprazole.

Formulations of the invention can be used for treatment of almost any physiological and/or psychological disorders for which the pharmaceutical compounds are indicated. Preferrably, the formulations provided herein are given to human patients in need of a therapy for disorders for which NSAIDs are typically indicated such as, for example, angina, aorto-pulmonary shunt occlusion, colorectal cancer, esophageal cancer, colon cancer, coronary artery disease, dementia, dysmenorrhea, myocardial infarction, rheumatoid arthritis, osteoarthritis, pain, headache, migraine headache, stroke, thrombocythemia, post-operative thromboembolism, ischemia, bursitis, cognative decline, fever, gout, musculoskelatal disorders, pericarditis and soft tissue injury.

In a particularly preferred embodiment, the NSAID employed in the formulation is aspirin at a dose between 25, and 150 mg, more preferably between 50 and 100 mg; the PPI is lansoprazole at a dose between 5mg and 100 mg, more preferably between 10 mg and 35 mg. This embodiment, is well suited for any of the well known cardiac, stroke, or other inflammatory conditions for which aspirin is currently prescribed.

Also provided is a method for protecting the gastrointestinal tract from the detrimental effects of NSAID therapy. In particular, the method comprises administering an NSAID in a single non-enterically coated dosage form that also comprises a PPI and a buffer.

### Examples

### Example 1

### PPI/NSAID Drug Substance Compatibility

Various NSAIDs were combined with lansoprazole with and without a buffer to determine the compatibility of the components. The various mixtures were placed at room temperature and 40°C with 75% relative humidity. The samples were tested initially and after two weeks at the storage conditions above. The results are reported in Table 1 below as a percent of the theoretical amount of a given component.

**Table 1**

| % of Theory | | | |
|---|---|---|---|
| Sample | Initial (lanso/NSAID) | Room Temp. (lanso/NSAID) | 40°C 75% humidity (lanso/NSAID) |
| Lansoprazole | 100.8/NA | 99.1/NA | 99.1/NA |
| Lansoprazole + Mg(OH)2 | 98.7/NA | --/-- | 97.9/NA |
| Lansoprazole + Piroxicam | 99.9/101.6 | 100.1/98.4 | 98.7/99.7 |
| Lansoprazole + Piroxicam + Mg(OH)2 | 100.0/101.3 | --/-- | 97.5/99.1 |
| Lansoprazole + Aspirin | 96.3/99.3 | 98.7/99.4 | 0/90.6 |
| Lansoprazole + Aspirin + Mg(OH)2 | 99.7/98.0 | --/-- | 86.8/67.7 |
| Lansoprazole + Naproxen | 99.5/99.4 | 100.0/100.0 | 98.6/100.7 |
| Lansoprazole + Naproxen + Mg(OH)2 | 99.0/98.9 | --/-- | 98.0/98.9 |
| Lansoprazole + Sodium Naproxen | 100.3/101.0 | 100.6/100.7 | 99.9/101.0 |
| Lansoprazole + Sodium Naproxen + Mg(OH)2 | 99.7/100.2 | --/-- | 98.8/99.8 |

As shown by the data in Table 1, the vast majority of the components were recovered after storage for two weeks, even under elevated temperature and humidity conditions.

### Example 2

### Substance Compatibility in Granulations and Tablets

In this example, granulations of a PPI and various NSAIDs were prepared to determine the compatibility of these substances with each other in a granulated form. Granulations containing the ingredients listed in Table 2 below were prepared.

**Table 2**

| Ingredient | A | B | C | D | E | F | G |
|---|---|---|---|---|---|---|---|
| Lansoprazole | 30 | 30 | 30 | 30 | -- | -- | -- |
| Aspirin | -- | 81 | -- | -- | 81 | -- | -- |
| Naproxen | -- | -- | 250 | -- | -- | 250 | -- |
| Piroxicam | -- | -- | -- | 20 | -- | -- | 20 |
| Magnesium Hydroxide | 145 | 145 | 145 | 145 | -- | -- | -- |
| Calcium Carbonate | 150 | 150 | 150 | 150 | -- | -- | -- |
| Mannitol | 150 | 150 | 150 | 150 | -- | -- | 50 |
| Avicel (microcrystalline Cellulose) | 75 | 75 | 75 | 75 | 150 | 100 | 50 |
| PVPP (Crosspovidone) | 35 | 35 | 35 | 35 | -- | 30 | -- |
| Wet mass with | water | water | water | water | * | 60% sucrose | 60% sucrose |
| Sieve mesh size | 20 | 20 | 20 | 20 | 40 | 20 | 20 |
| Drying Temp. °C (overnight) | 45 | 45 | 45** | 45 | * | 45 | 45 |

| | | | | | | | |
|---|---|---|---|---|---|---|---|
| * The dry powders were mixed and directly compressed with a Carver press to hold together. These slugs were then pressed through a 40 mesh screen. ** Granulation C was pressed through a 10 mesh screen after drying. | | | | | | | |

The potency (mg/g) of each drug substance in each granulation was determined by HPLC. The potency data is presented in Table 3, below.

**Table 3**

| Granulation | Initial Potency (mg/g) Lanso/NSAID |
|---|---|
| A | 51.1/-- |
| B | 44.7/47.0 |
| C | 36.4/304.1 |
| D | 48.9/33.7 |
| E | --/409.6 |
| F | --/425.0 |
| G | --/125.4 |

Granulations A, C, and D, as well as naproxen and piroxicam drug substances were weighed and separately placed in scintillation vials and stored at room temperature (RT) and at 40°C with 75% to 85% relative humidity ("Extreme Conditions"). Granulations A, C, and D, as well as the naproxen and piroxicam, were tested for potency of the lansoprazole and NSAID after 19 and 33 days at the room temperature conditions and after two and four weeks under the Extreme Conditions. The results (given in mg/g) of the compatibility assessment at the various conditions is shown in Table 4, below.

**Table 4**

| Sample | 19 Days RT (Lanso/NSAID) | 2 Weeks Extreme Condtions (Lanso/NSAID) | 33 Days RT (Lanso/NSAID) | 4 Weeks Extreme Conditions (Lanso/NSAID) |
|---|---|---|---|---|
| Granulation A | 51.9/NA | 51.9/NA | 52.1/NA | 51.2/NA |
| Granulation C | 36.4/294.2 | 36.5/307.9 | 36.3/296.3 | 36.0/304.4 |
| Granulation D | 48.9/32.5 | 49.4/32.8 | 49.5/32.7 | 48.2/32.8 |
| Naproxen | NA/997.2 | NA/996.7 | NA/998.2 | NA/1000.9 |
| Piroxicam | NA/1027.1 | NA/942.2 | NA/998.5 | NA/1005.5 |

### Example 3

### PPI, NSAID, Buffered Tablets and Their Dissolution

Tablets were made using granulation A (from Example 2) in combination with granulations E, F, or G from Example 2. The granules were weighed in such a manner as to make tablets that contained 30 mg of lansoprazole and either 81 mg of aspirin, 250 mg of naproxen, or 20 mg of piroxicam. The components were compressed into tablets after mixing the different granules. The granules were compressed into tablets at ¼ to ½ metric ton of pressure to form a tablet that would rapidly release its components upon dissolution.

The dissolution experiments were performed using the following reagents and conditions. The vessels were sampled using 5-mL disposable syringes with stainless steel cannulae and then filtered through 0.45-µm PTFE or PVDF syringe filters (Gelman Acrodisc) prior to HPLC analysis. Dissolution was facilitated with a Van Kel VK700 mixing apparatus equipped with two paddles turning at 75 rpm. The contents of each dissolution vessel is given below in Table 5.

**Table 5**

| | **Medium Volume mL** | **Dissolution Medium at 37°C** |
|---|---|---|
| **A + E** | 500 | 0.05M, pH 4.5 acetate buffer (USP medium) |
| **A + G** | 900 | 0.1N HCl (USP medium) |
| **A + F** | 900 | 0.1M, pH 7.4 phosphate buffer (USP medium) |

Samples from the beakers were taken after 5, 15, 30, 45, and 60 minutes and filtered prior to HPLC analysis. The percent of the total NSAID or PPI (lansoprazole) as a function of time is shown in Table 5 below.

**Table 6**

| Tablet Granulations | % Release 5 Minutes (lanso/NSAID) | % Release 15 Minutes (lanso/NSAID) | % Release 30 Minutes (lanso/NSAID) | % Release 45 Minutes (lanso/NSAID) | % Release 60 Minutes (lanso/NSAID |
|---|---|---|---|---|---|
| A + E | 47.0/79.3 | 70.0/93.7 | 78.0/94.0 | 80.0/93.0 | 81.0/96.0 |
| A + F | 46.2/49.3 | 80.2/96.3 | 86.2/100.3 | 87.7/101.3 | 87.7/101.3 |
| A + G | 48.0/21.2 | 74.0/46.6 | 81.0/69.8 | 84.0/82.8 | 84.0/87.0 |

As shown by Table 6 the majority of the various components in each of the tablets were recovered upon dissolution.

While the invention has been described above with respect to the preferred embodiments, it will be understood that the invention is adapted to numerous rearrangements, modifications, and alterations, and all such arrangements, modifications, and alterations are intended to be within the scope of the appended claims.

## Claims

1. A non-enterically coated dosage form comprising:
a) a proton pump inhibitor;
b) a buffer; and
c) a non-steroidal anti-inflammatory drug.

2. The dosage form of claim 1 wherein the buffer is selected from the group consisting of a water soluble buffer, a water insoluble buffer, and a combination of a water soluble buffer and a water insoluble buffer.

3. The dosage form of claim 1 wherein the non-steroidal anti-inflammatory drug is aspirin.

4. The dosage form of claim 3 wherein the proton pump inhibitor is lansoprazole.

5. The dosage form of claim 4 wherein the dosage form comprises aspirin at an amount between 50 mg and 100 mg.

6. Use of a non-enterically coated dosage form comprising a buffer, a proton pump inhibitor, and a non-steroidal anti-inflammatory drug for manufacturing a medicament for treating a condition selected from the group consisting of angina, aorto- pulmonary shunt occlusion, colorectal cancer, esophageal cancer, colon cancer, coronary artery disease, dementia, dysmenorrhea, myocardial infarction, rheumatoid arthritis, osteoarthritis, pain, headache, migraine headache, stroke, thrombocythemia, post-operative thrombeoembolism, ischemia, bursitis, cognative decline, fever, gout, musculoskelatal disorders, soft tissue injury, and pericarditis by administration to a patient having one or more of the above conditions.

7. Use of a NSAID in combination with a buffer and a Proton pump inhibitor PPI, wherein the NSAID, PPI and buffer comprise a single non-enterically coated dosage form, for manufacturing a medicament for protecting the gastrointestinal tract from NSAID therapy.

## Patentansprüche

1. Eine nicht Magensaft-resistent beschichtete Dosierform, die folgendes umfaßt:
a) einen Protonen-Pumpen-Inhibitor;
b) einen Puffer und
c) ein nichtsteroidales Antirheumatikum.

2. Die Dosierform von Anspruch 1, worin der Puffer gewählt ist aus der Gruppe bestehend aus einem wasserlöslichen Puffer, einem wasserunlöslichen Puffer und einer Kombination aus einem wasserlöslichen Puffer und einem wasserunlöslichen Puffer.

3. Die Doszerform von Anspruch 1, worin das nichtsteroidale Antirheumatikum Aspirin ist.

4. Die Dosierform von Anspruch 3, worin der Protonen-Pumpen-Inhibitor Lansoprazol ist.

5. Die Dosierform von Anspruch 4, worin die Dosierform Aspirin in einer Menge zwischen 50 mg und 100 mg umfaßt.

6. Verwendung einer nicht Magensaft-resistent beschichteten Dosierform, die folgendes umfaßt; einen Puffer, einen Protonen-Pumpen-Inhibitor und ein nichtsteroidales Antirheumatikum zur Herstellung eines Medikaments zur Behandlung eines Leidens, gewählt aus der Gruppe bestehend aus Angina, aorto-pulmonalem Shunt-Verschluß, Kolorektalkrebs, Speiseröhrenkrebs, Kolonkrebs, Koronararterien-Verschlußkrankheit, Demenz, Dysmenorrhoe, Myokardinfarkt, Rheumatoidarthritis, Osteoarthritis, Schmerz, Kopfschmerz, Migräne-Kopfschmerz, Schlaganfall, Thrombozythämie, postoperativem Thromboembolismus, Ischämie, Bursitis, kognitive Verschlechterung, Fieber, Gicht, Muskel-Skelett-Erkrankungen, Weichteil-Verletzung und Perikarditis, durch Verabreichung an einen Patienten, der eines oder mehrere der obengenannten Leiden hat.

7. Verwendung eines NSAID (nichtsteroidalen Antirheumatikums) in Kombination mit einem Puffer und einem Protonen-Pumpen-Inhibitor (PPI), worin das NSAID, der PPI und der Puffer eine einzelne nicht Magensaft-resistent beschichtete Dosierform umfassen, zur Herstellung eines Medikaments zum Schutz des Gastrointestinaltrakts vor der NSAID-Therapie.

## Revendications

1. Forme posologique non gastrorésistante comprenant :
a) un inhibiteur de pompe à protons;
b) un tampon ; et
c) un médicament anti-inflammatoire non stéroïdien.

2. Forme posologique selon la revendication 1, dans laquelle le tampon est choisi dans le groupe constitué par un tampon soluble dans l'eau, un tampon insoluble dans l'eau et une combinaison d'un tampon soluble dans l'eau et d'un tampon insoluble dans l'eau.

3. Forme posologique selon la revendication 1, dans laquelle le médicament anti-inflammatoire non stéroïdien est l'aspirine.

4. Forme posologique selon la revendication 3, dans laquelle l'inhibiteur de pompe à protons et le lansoprazole.

5. Forme posologique selon la revendication 4, dans laquelle la forme posologique comprend de l'aspirine dans une quantité comprise entre 50 mg et 100 mg.

6. Utilisation d'une forme posologique non gastrorésistante comprenant un tampon, un inhibiteur de pompe à protons et un médicament anti-inflammatoire non stéroïdien pour fabriquer un médicament destiné à traiter une affection choisie dans le groupe constitué par l'angine, une occlusion de court-circuit aorto-pulmonaire, le cancer colorectal, le cancer de l'oesophage, le cancer du côlon, la maladie coronarienne, la démence, la dysménorrhée, un infarctus du myocarde, la polyarthrite rhumatoïde, l'arthrose, une douleur, un mal de tête, un mal de tête de type migraine, un accident vasculaire cérébral, la thrombocytémie, la thromboembolie post-opératoire, l'ischémie, la bursite, le déclin cognitif, la fièvre, la goutte, les troubles musculo-squelettiques, une lésion des tissus mous et une péricardite par administration à un patient atteint d'une ou plusieurs des affections précédentes.

7. Utilisation d'un AINS en combinaison avec un tampon et un inhibiteur de pompe à protons IPP, dans laquelle l'AINS, l'IPP et le tampon comprennent une forme posologique non gastrorésistante simple, pour fabriquer un médicament destiné à protéger le tractus gastrointestinal lors d'un traitement aux AINS.
